(19) Europäisches Patentamt
European Patent Office
Office européen des brevets

(11) EP 0 897 923 A1

(12) EUROPEAN PATENT APPLICATION
published in accordance with Art. 158(3) EPC

(43) Date of publication:
24.02.1999 Bulletin 1999/08

(21) Application number: 97917409.1

(22) Date of filing: 17.04.1997

(51) Int. Cl.[6]: C07D 455/04, A61K 31/435

(86) International application number:
PCT/JP97/01320

(87) International publication number:
WO 97/40046 (30.10.1997 Gazette 1997/46)

(84) Designated Contracting States:
AT BE CH DE DK ES FI FR GB GR IE IT LI LU MC NL PT SE

(30) Priority: 19.04.1996 JP 97863/96

(71) Applicant:
NIPPON SHINYAKU COMPANY, LIMITED
Minami-ku Kyoto-shi Kyoto 601 (JP)

(72) Inventors:
- YAMAMOTO, Osamu
  Kyoto 606 (JP)
- SHIROUCHI, Yoshiaki
  Kyoto 619-02 (JP)

(74) Representative:
Strych, Werner Maximilian Josef, Dr. et al
Hansmann & Vogeser,
Patent- und Rechtsanwälte,
Albert-Rosshaupter-Strasse 65
81369 München (DE)

# (54) BENZOQUINOLIZINE DERIVATIVES AND MEDICINAL COMPOSITIONS

(57) The invention is directed to a benzoquinolizine derivative of the following formula [1], its pharmaceutically acceptable salt, and a pharmaceutical composition comprising the said compound,

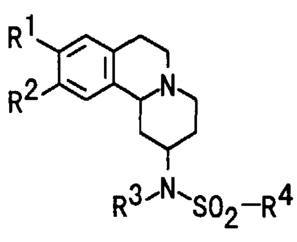

[1]

wherein $R^1$ represents hydroxy or alkoxy; $R^2$ represents hydrogen, hydroxy or alkoxy; $R^3$ represents alkyl; the said alkyl is optionally substituted by hydroxy, alkoxy, amino, monoalkylamino, or dialkylamino; $R^4$ represents alkyl (the said alkyl is optionally substituted by hydroxy or alkoxy), aryl (the said aryl is optionally substituted by alkyl, halogen, hydroxy, alkoxy, amino, monoalkylamino, or dialkylamino), monoalkylamino, or dialkylamino.

The compound of the invention has gastrointestinal motility modulating activity and is effective in the treatment of irritable bowel syndrome.

EP 0 897 923 A1

## Description

TECHNICAL FIELD

The present invention relates to a novel benzoquinolizine derivative and its pharmaceutically acceptable salt.

The compound of the invention has gastrointestinal motility modulating activity and is useful for the treatment of irritable bowel syndrome.

BACKGROUND ART

Irritable bowel syndrome (IBS) is a functional bowel disease characterized by chronic altered bowel habits (constipation, diarrhea, and alternating diarrhea and constipation) and abdominal pain as a cardinal manifestation with or without other abdominal symptoms such as abdominal distention or discomfort and borborygmus, and is pathophysiologically considered to be a dyskinesia of the intestinal tract. As the pharmacotherapy of this disease, anticholinergic drugs acting against the movement of the gastrointestinal tract and trimebutine having gastrointestinal motility modulating activity are in use but there has been a standing demand for development of a more effective new drug.

Japanese Laid-open S62-67031 (corresponding to USP 4,673,680) mentions that a benzoquinolizine derivative has gastrointestinal peristalsis modulating activity and is useful for the treatment of irritable bowel syndrome among other diseases. However, as will be apparent from the test examples which appear hereinafter, those species which resemble the compound of the present invention among compounds disclosed in the patent are not possessed of gastrointestinal peristalsis modulating activity and, therefore, cannot be applied to the therapy of irritable bowel syndrome with success.

In addition, USP 4,076,820 describes a benzoquinolizine derivative of the following formula [2a],

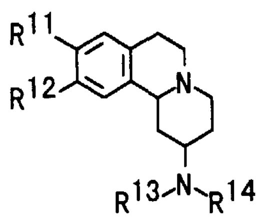

[2a]

wherein $R^{11}$ and $R^{12}$ are the same or different and each represents hydrogen, lower alkyl, lower alkoxy, or halogen; $R^{13}$ represents hydrogen, lower alkyl, or aryl; $R^{14}$ represents $-SO_2R^{15}$, $-CONH_2$, or $-CXNHR^{16}$; $R^{15}$ represents lower alkyl or aryl; X represents oxygen or sulfur; $R^{16}$ represents aryl or aryl carbonyl.

Among species of the compound of the present invention, of the formula [1] presented hereinafter, those in which $R^1$ is alkoxy; $R^2$ is hydrogen or alkoxy; $R^3$ is unsubstituted alkyl; and $R^4$ is (1) unsubstituted alkyl or (2) phenyl which is optionally substituted by alkyl, halogen, alkoxy, amino, monoalkylamino, or dialkylamino fall within the scope of the claims of the above-mentioned USP 4,076,820 but none of those compounds are specifically disclosed in the said USP 4,076,820.

In addition, the said USP 4,076,820 mentions that a compound of the formula [2a] has hypotensive activity but does not indicate that it ever has gastrointestinal motility modulating activity and is useful for the treatment of irritable bowel syndrome.

Furthermore, USP 4,481,200 describes a benzoquinolizine derivative of the following formula [2b],

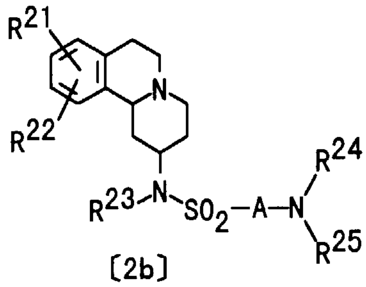

[2b]

wherein $R^{21}$ and $R^{22}$ are the same or different and each represents hydrogen, lower alkyl, lower alkoxy, or halogen; $R^{23}$ represents methyl or ethyl; A represents a bond between S and N atoms or an alkylene group of 1-3 carbon atoms; $R^{24}$ and $R^{25}$ each independently represents hydrogen, lower alkyl, aryl, or aryl-lower-alkyl, or $R^{24}$ and $R^{25}$ taken together with the adjacent N represent a 5-membered or 6-membered heterocyclic group.

Among species of the compound of the present invention, of the formula [1] presented hereinafter, those in which $R^1$ is alkoxy; $R^2$ is hydrogen or alkoxy; $R^3$ is unsubstituted alkyl; $R^4$ is $-NR^5R^6$; $R^5$ and $R^6$ are the same or different and each is alkyl, or $R^5$ and $R^6$ taken together with the adjacent N atom represent a 5-membered or 6-membered cyclic amino group fall within the scope of the claims of the above-mentioned USP 4,481,200 but none of those compounds are specifically described in the said USP 4,481,200.

The said USP 4,481,200 mentions that the compound of the formula [2b] has antidepressant, antidiabetic, and platelet aggregation inhibitory activities but does not describe that the compound ever has gastrointestinal motility modulating activity and is useful for the treatment of irritable bowel syndrome.

DISCLOSURE OF THE INVENTION

The object of the present invention is to provide a compound which has superior gastrointestinal motility modulating activity and is useful as a therapeutic drug for irritable bowel syndrome.

The inventors of the present invention found that a benzoquinolizine derivative of the following formula [1] and its pharmaceutically acceptable salt are useful for accomplishing the above object and thereby have completed the present invention,

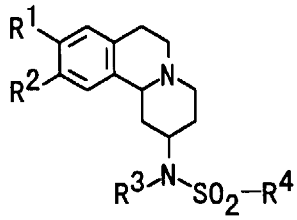

[1]

wherein

$R^1$ represents hydroxy or alkoxy;
$R^2$ represents hydrogen, hydroxy or alkoxy;
$R^3$ represents alkyl; the said alkyl is optionally substituted by hydroxy, alkoxy, amino, monoalkylamino, or dialkylamino;
$R^4$ represents alkyl (the said alkyl is optionally substituted by hydroxy or alkoxy), aryl (the said aryl is optionally substituted by alkyl, halogen, hydroxy, alkoxy, amino, monoalkylamino, or dialkylamino), or $-NR^5R^6$; $R^5$ and $R^6$ are the same or different and each represents alkyl, or $R^5$ and $R^6$ taken together with the adjacent N atom represent a 5-membered or 6-membered cyclic amino group.

The compound of the invention is a novel compound not described heretofore in literatures. The compound of the invention has very superior gastrointestinal motility modulating activity and can be applied to the treatment of irritable bowel syndrome. This is one of the features of the invention.

The present invention has been completed for the first time on the basis of that the inventors have found that the compound exhibits a specific pharmacological activity of its own.

The invention is now described in detail.

In the context of the present invention, the "alkyl" or the alkyl moiety of said monoalkylamino or dialkylamino includes straight-chain or branched-chain alkyl groups containing 1-7 carbon atoms, such as methyl, ethyl, n-propyl, isopropyl, n-butyl, isobutyl, sec-butyl, tert-butyl, n-pentyl, isopentyl, tert-pentyl, n-hexyl, isohexyl, n-heptyl, and isoheptyl.

The "alkoxy" includes straight-chain or branched-chain alkoxy groups containing 1-7 carbon atoms, such as methoxy, ethoxy, n-propoxy, isopropoxy, n-butoxy, isobutoxy, sec-butoxy, tert-butoxy, n-pentyloxy, isopentyloxy, tert-pentyloxy, n-hexyloxy, isohexyloxy, n-heptyloxy, and isoheptyloxy.

The "aryl" includes aryl groups containing 6-10 carbon atoms, such as phenyl, 1-naphthyl, and 2-naphthyl.

The "halogen" means fluorine, chlorine, bromine, or iodine.

The alkyl moiety of said monoalkylamino or dialkylamino includes the alkyl groups mentioned above.

The 5-membered or 6-membered cyclic amino group may contain one nitrogen, oxygen, or sulfur atom as atoms constructing the ring, thus including pyrrolidino, piperidino, morpholino, thiomorpholino, and piperazino.

The preferred species of the compound of the invention are such that the hydrogen atom at 2-position and the hydrogen atom at 11b-position are in the steric relationship of cis; $R^1$ is alkoxy; $R^2$ is hydrogen; $R^3$ is unsubstituted alkyl; $R^4$ is (1) unsubstituted alkyl, (2) aryl which is optionally substituted by halogen or alkoxy, or (3) dialkylamino.

The particularly preferred species of the compound of the invention are such that the configuration of the carbon atom in 2-position is R, the configuration of the carbon atom in 11b-position is S; $R^1$ is alkoxy; $R^2$ is hydrogen; $R^3$ is unsubstituted alkyl; $R^4$ is (1) unsubstituted alkyl, (2) aryl which is optionally substituted by halogen or alkoxy, or (3) dialkylamino.

The following is a partial listing of the preferred species of the compound of the invention.

(1) N-methyl-N-[(2RS,11bSR)-9-methoxy-1,3,4,6,7,11b-hexahydro-2H-benzo[a]quinolizin-2-yl]methanesulfonamide

(2) N-methyl-N-[(2RS,11bSR)-9-methoxy-1,3,4,6,7,11b-hexahydro-2H-benzo[a]quinolizin-2-yl]-4-methoxy benzenesulfonamide

(3) N-methyl-N-[(2RS,11bSR)-9-methoxy-1,3,4,6,7,11b-hexahydro-2H-benzo[a]quinolizin-2-yl]benzenesulfonamide

(4) N-methyl-N-[(2RS,11bSR)-9-methoxy-1,3,4,6,7,11b-hexahydro-2H-benzo[a]quinolizin-2-yl]-4-fluorobenzensulfonamide

(5) N-methyl-N-[(2RS,11bSR)-9-methoxy-1,3,4,6,7,11b-hexahydro-2H-benzo[a]quinolizin-2-yl]-2,5-dimethoxybenzenesulfonamide

(6) N,N-dimethyl-N'-[(2RS,11bSR)-9-methoxy-1,3,4,6,7,11b-hexahydro-2H-benzo[a]quinolizin-2-yl]-N'-methylsulfamide

(7) N-methyl-N-[(2RS,11bSR)-9-methoxy-1,3,4,6,7,11b-hexahydro-2H-benzo[a]quinolizin-2-yl]ethanesulfonamide

(8) N-ethyl-N-[(2RS,11bSR)-9-methoxy-1,3,4,6,7,11b-hexahydro-2H-benzo[a]quinolizin-2-yl]methanesulfonamide

The compound [1] of the invention can be produced typically in accordance with the following reaction scheme,

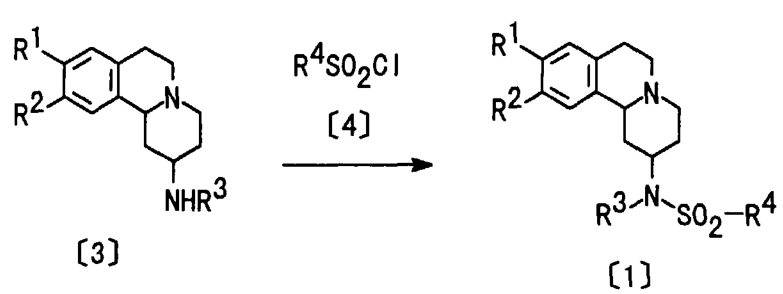


[3] [1]

wherein $R^1$, $R^2$, $R^3$, and $R^4$ have the same meanings as defined hereinbefore.

The compound [1] of the invention can be produced by reacting compound [3] with compound [4].

This reaction is generally carried out in a suitable solvent [e.g. polar solvents such as acetonitrile and N,N-dimethylformamide (DMF); ethers such as tetrahydrofuran (THF) and diethyl ether; halogenated hydrocarbons such as chloroform and methylene chloride; esters such as methyl acetate and ethyl acetate; hydrocarbons such as benzene,

toluene and n-hexane; or mixtures of said solvents] in the presence of a base [e.g. inorganic bases such as potassium carbonate, sodium carbonate and sodium hydrogen carbonate; and organic bases such as pyridine, 4-dimethylaminopyridine and triethylamine] at -20 to 150°C. The reaction time is dependent on the species of compound [3] and compound [4] and the reaction temperature but preferably may range from 30 minutes to 100 hours. The amount of compound [4] is preferably 1-2.0 molar equivalents relative to compound [3].

In the above production of the compound of the invention, in cases where the starting compound has a substituent group (e.g. hydroxy) which should not be involved in the reaction, it is generally preferable to protect the compound with a protective group (for hydroxy, it may for example be methyl, methoxymethyl, benzyl, acetyl or tert-butoxycarbonyl) prior to conducting the intended reaction. After the reaction, the protective group can be removed by a known procedure such as catalytic reduction, alkali treatment, acid treatment, or acid treatment subsequent to reaction with boron trifluoride.

For example, the compound [1a] of the invention in which $R^1$ is hydroxy can be produced by the following reaction,

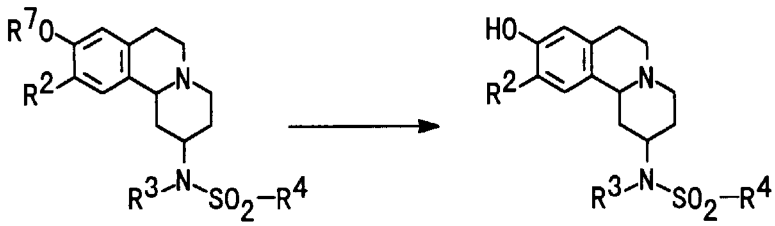

[5] [1a]

wherein $R^2$, $R^3$, and $R^4$ have the same meanings as defined hereinbefore; $R^7$ represents alkyl.

The compound [1a] can be produced by subjecting the compound [5] obtained by the above method for production of the compound of the invention to cleavage reaction of the aryl ether.

For example, a boron trihalide (e.g. boron tribromide, boron trichloride, or boron triiodide) is added dropwise to compound [5] in an inert solvent such as methylene chloride at 0°C to room temperature and the reaction is allowed to proceed for 8-24 hours. After the completion of the reaction, water or diluted hydrochloric acid is added to the reaction mixture for hydrolysis of the boron complex to give compound [1a].

The cleavage reaction of the aryl ether can also be carried out by the method using hydrogen bromide, hydrogen iodide, trifluoroacetic acid, aluminum chloride, aluminum bromide or the like, the method using an organometal reagent such as a Grignard reagent, or the method using a base such as sodium thiolate.

The compound [1b] of the invention which corresponds to $R^1$ is alkoxy can be produced by the following process as well,

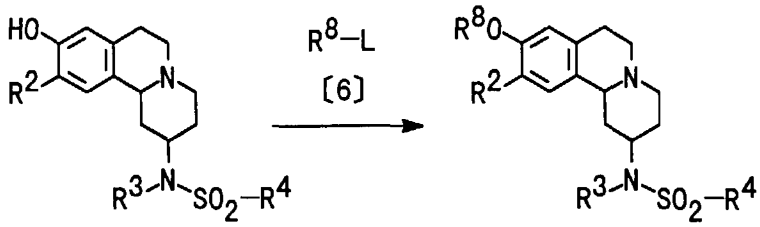


[1a] [1b]

wherein $R^2$, $R^3$, and $R^4$ have the same meanings as defined hereinbefore; $R^8$ represents alkyl; L represents a leaving group such as halogen.

The compound [1b] can be produced by alkylating the compound [1a] obtained by the above method for production of the compound of the invention.

For example, compound [1b] can be produced by reacting compound [1a] with an alkyl halide [6] in the presence of a base (e.g. sodium amide, potassium carbonate, triethylamine, sodium hydride, sodium hydroxide, etc.) in an inert solvent (e.g. N,N-dimethylformamide, dimethyl sulfoxide, dimethoxyethane or tetrahydrofuran) at 0 to 100°C.

The compound [3], compound [4], and compound [6] which are used as starting compounds in the above reactions are either known compounds or can be produced in accordance with the known processes as described in Reference Examples.

Because the carbon atoms in the 2- and 11b-positions are asymmetric carbon atoms, the compound of the invention may exist in 4 different optically active substances. Thus, the optically active substances are the (2R,11bR)-substance, (2S,11bS)-substance, (2R,11bS)-substance, and (2S,11bR)-substance. The (2R,11bS)-substance and (2S,11bR)-substance are such that the hydrogen atom at 2-position and the hydrogen atom at 11b-postion are in the steric relationship of cis and the (2R,11bR) - and (2S, 11bS)-substance are such that said steric relationship is trans. Those respective optical active substances and mixtures thereof also fall within the scope of the invention.

The cis- and trans-substances can be respectively isolated from their mixture by the conventional column chromatography.

Each optically active substance can be isolated by liquid chromatography using a chiral column (e.g. CHIRALCEL OD and CHIRALCEL OF, made by Daicel Chemical Industries, Ltd.). As an alternative, each optically active substance can be synthesized by using compound [3] of the corresponding optically active form as a starting compound.

The compound of the invention can be provided in the form of a pharmaceutically acceptable salt by the per se known method. The salt includes salts with mineral acids such as hydrochloric acid, hydrobromic acid, sulfuric acid and phosphoric acid; and salts with organic acids such as acetic acid, citric acid, tartaric acid, maleic acid, succinic acid, fumaric acid, p-toluenesulfonic acid, benzenesulfonic acid and methanesulfonic acid. Taking the hydrochloride as an example, it can be obtained by dissolving the compound of the invention in an alcoholic solution of hydrogen chloride.

From said reaction mixture, the compound of the invention or the salt thereof can be isolated and purified by the conventional isolation and purification techniques such as extraction, concentration, neutralization, filtration, recrystallization, column chromatography, and thin-layer chromatography.

As will be shown hereinafter, the toxicity of the compound of the invention is not high.

For use of the compound of the invention as a drug, the compound of the invention is administered to an animal inclusive of man either as it is or in the form of a pharmaceutical composition typically containing 0.1-99.5%, preferably 0.5-90%, of the compound in a pharmaceutically acceptable, nontoxic and inert carrier.

As the carrier, one or more different solid, semisolid, or liquid diluents, fillers, and/or other formulation auxiliaries are employed. The pharmaceutical composition is preferably administered in unit dosage forms. The pharmaceutical composition of invention can be administered intravenously, orally, into tissues, topically (e.g. transdermally), or rectally. Of course, a dosage form suited to each such route of administration should be selectively used. Oral administration is particularly preferred.

The dosage of such a pharmaceutical composition for the treatment of irritable bowel syndrome is preferably established with reference to the patient's factors such as age and body weight, the route of administration, and the nature and severity of illness. The usual dosage for an adult human in terms of the compound of the invention is generally 0.3-300 mg/day/patient and preferably 3-30 mg/day/patient. Low doses may be sufficient in some cases and higher doses is optionally needed in other cases.

Moreover, the above daily dosage is optionally administered in 2-3 divided doses.

BEST MODE FOR CARRYING OUT THE INVENTION

The following reference examples, working examples, test examples, and formulation examples are intended to describe the present invention in further detail.

Reference Example 1

N-(9-Methoxy-1,3,4,6,7,11b-hexahydro-2H-benzo[a]quinolizin-2-yl)-N-methylamine

Step 1

N-(3-Methoxyphenethyl)formamide

A solution of 100 g of 3-methoxyphenethylamine in 200 ml of ethyl formate was refluxed for 5 hours. This reaction mixture was concentrated under reduced pressure and the residue was purified by silica gel column chromatography (10% methanol-chloroform) to provide 115 g of the title compound.

Step 2

6-Methoxy-3,4-dihydroisoquinoline

To 80 g of polyphosphoric acid preheated at 145°C was added 20.0 g of N-(3-methoxyphenethyl)formamide. The mixture was stirred at 145°C for 3 hours, at the end of which time it was poured onto ice and made alkaline with 2N-NaOH. This aqueous solution was extracted with chloroform three times and the extract was dried over magnesium sulfate, filtered, and concentrated. The residue was purified by silica gel column chromatography (5% methanol-chloroform) to provide 15.0 g of the title compound.

Step 3

9-Methoxy-1,3,4,6,7,11b-hexahydro-2H-benzo[a]quinolizin-2-one

A solution of 25.0 g of 6-methoxy-3,4-dihydroisoquinoline in 50 ml of methyl vinyl ketone was refluxed for 3 hours. This reaction mixture was concentrated under reduced pressure and the residue was purified by silica gel column chromatography (5% methanol-chloroform) to provide 13.0 g of the title compound.

Step 4

N-(9-Methoxy-1,3,4,6,7,11b-hexahydro-2H-benzo[a]quinolizin-2-yl)-N-methylamine

To a solution of 10.0 g of 9-methoxy-1,3,4,6,7,11b-hexahydro-2H-benzo[a]quinolizin-2-one in 200 ml of a methanol was added 20 ml of 40% methylamine-methanol under ice-cooling and stirring. Then, 5.0 g of sodium cyanoborohydride and 20 ml of acetic acid were added and the mixture was stirred at room temperature for 4 hours. This reaction mixture was concentrated under reduced pressure and the residue was diluted with water, made alkaline with 2N-NaOH, and extracted with chloroform three times. The extract was dried over magnesium sulfate, filtered, and concentrated. The residue was purified by silica gel column chromatography (chloroform:methanol:aqueous ammonia = 100:10:1) to provide 8.2 g of the title compound.

The compound thus obtained is a mixture of compounds in which the steric relationship of hydrogen at 2-position and hydrogen at 11b-position are cis and trans, respectively.

The following compounds were produced generally in the same manner as Reference Example 1.

N-(9-Methoxy-1,3,4,6,7,11b-hexahydro-2H-benzo[a]quinolizin-2-yl)amine
N-(9-Methoxy-1,3,4,6,7,11b-hexahydro-2H-benzo[a]quinolizin-2-yl)-N-(2-methoxyethyl)amine
N-(9-Methoxy-1,3,4,6,7,11b-hexahydro-2H-benzo[a]quinolizin-2-yl)-N-isopropylamine
N-(9-Methoxy-1,3,4,6,7,11b-hexahydro-2H-benzo[a]quinolizin-2-yl)-N-ethylamine

Reference Example 2

N-[(2R,11bS)-9-Methoxy-1,3,4,6,7,11b-hexahydro-2H-benzo[a]quinolizin-2-yl]-N-methylamine

Step 1

11bS-9-Methoxy-1,3,4,6,7,11b-hexahydro-2H-benzo[a]quinolizin-2-one

A solution of 5.0 g of the 9-methoxy-1,3,4,6,7,11b-hexahydro-2H-benzo[a]quinolizin-2-one obtained in Step 3 of Reference Example 1 in 135 ml of ethyl acetate was mixed with a solution of 8.35g of (-)-di-p-toluoyl-L-tartaric acid in 135 ml of ethyl acetate and the mixture was stirred at 50°C for 5 hours and then at room temperature for 2 hours. The fine crystalline precipitate of the produced tartrate was collected by filtration and after addition of water, the mixture was made alkaline with 2N-NaOH. This solution was extracted with chloroform three times and the extract was dried over magnesium sulfate and concentrated. The residue was recrystallized from n-hexane/ethyl acetate to provide 3.2 g of the title compound.

Specific rotation $[\alpha]_D^{25}$ = -107.39° (c=1.028 g/dl, $CHCl_3$)

Step 2

N-[(2R,11bS)-9-Methoxy-1,3-4,6-7,11b-hexahydro-2H-benzo[a]quinolizin-2-yl]-N-methylamine

To a solution of 5.0 g of the 11bS-9-methoxy-1,3,4,6,7,11b-hexahydro-2H-benzo[a]quinolizin-2-one obtained in Step 1 in 60 ml of benzene was added 70 ml of 2M-methylamine/tetrahydrofuran, and a solution of 2.05 g of titanium tetrachloride in 10 ml of benzene was added dropwise over 30 minutes under ice-cooling and stirring and then further stirred at room temperature for 2 hours. This reaction mixture was filtered with the aid of Celite and washed with benzene-ether (=2:1). The filtrate and washes were combined and concentrated. The residue was dissolved in 100 ml of ethanol. To this solution was added 0.82 g of sodium borohydride in small portions under ice-cooling and stirring and the mixture was further stirred at room temperature for 16 hours. After addition of a small amount of ice, the mixture was concentrated, diluted with water, made alkaline with 2N-NaOH, and extracted with chloroform three times. The extract was dried over magnesium sulfate and concentrated to provide 5.3 g of a syrupy substance [(2R,11bS)-substance:(2S,11bS)-substance = 9:1].

Reference Example 3

N-[(2R,11bS)-9-Methoxy-1,3,4,6,7,11b-hexahydro-2H-benzo[a]quinolizin-2-yl]-N-ethylamine

To a solution of 4.0 g of the 11bS-9-methoxy-1,3,4,6,7,11b-hexahydro-2H-benzo[a]quinolizin-2-one obtained in Step 1 of Reference Example 2 in 80 ml of methanol were added 4 ml of 70% aqueous ethylamine and 0.4 g of platinum oxide, and the catalytic reduction was carried out for 2 hours. The reaction mixture was then filtered and concentrated to provide 4.2 g of a syrupy substance [(2R,11bS)-substance:(2S,11bS)-substance = 8:2].

Example 1

N-Methyl-N-[(2RS,11bSR)-9-methoxy-1,3,4,6,7,11b-hexahydro-2H-benzo[a]quinolizin-2-yl]methanesulfonamide hydrochloride (Compound No. 1)

To a solution of 2.0 g of the N-(9-methoxy-1,3,4,6,7,11b-hexahydro-2H-benzo[a]quinolizin-2-yl)-N-methylamine obtained in Reference Example 1 in 40 ml of methylene chloride was added 2.7 g of methanesulfonyl chloride, followed by addition of 2.5 g of triethylamine, under ice-cooling and stirring and the mixture was stirred at room temperature for 6 hours. This reaction mixture was diluted with water, made alkaline with 2N-NaOH, and extracted with chloroform three times. The extract was dried over magnesium sulfate, filtered, and concentrated to give a residue [a cis/trans mixture]. This residue was purified by silica gel column chromatography (5% methanol-chloroform) to provide the cis substance. This compound was dissolved in 5 ml of methanol, treated with 10 ml of 20% HCl/methanol, and concentrated. The residue was recrystallized from methanol to provide 1.1 g of the title compound.

Example 2

N-Methyl-N-[(2RS,11bSR)-9-methoxy-1,3,4,6,7,11b-hexahydro-2H-benzo[a]quinolizin-2-yl]-4-methoxybenzenesulfonamide hydrochloride (Compound No. 6)

To a solution of 7.9 g of the N-(9-methoxy-1,3,4,6,7,11b-hexahydro-2H-benzo[a]quinolizin-2-yl)-N-methylamine obtained in Reference Example 1 in 160 ml of methylene chloride was added 9.9 g of 4-methoxybenzenesulfonyl chloride, followed by addition of 4.9 g of triethylamine, under ice-cooling and stirring, and the mixture was stirred at room temperature for 6 hours. This reaction mixture was diluted with water, made alkaline with 2N-NaOH, and extracted with chloroform three times. The extract was dried over magnesium sulfate, filtered, and concentrated to give a residue (a cis/trans mixture). This residue was purified by silica gel column chromatography (5% methanol-chloroform) to provide the cis-substance. This compound was dissolved in 10 ml of methanol, 20 ml of 20% hydrogen chloride/methanol was added, and the mixture was concentrated. The residue was recrystallized from methanol to provide 3.7 g of the title compound.

Example 3

N-Methyl-N-[(2RS,11bSR)-9-hydroxy-1,3,4,6,7,11b-hexahydro-2H-benzo[a]quinolizin-2-yl]methanesulfonamide hydrochloride (Compound No. 9)

To a solution of 1.20 g of the N-methyl-N-[(2RS,11bSR)-9-methoxy-1,3,4,6,7,11b-hexahydro-2H-benzo[a]quinolizin-2-yl]methanesulfonamide obtained in Example 1 in 30 ml of methylene chloride was added a solution containing 0.7 ml of boron tribromide in 5 ml of methylene chloride gradually dropwise under ice-cooling. The mixture was stirred at room temperature for 3 hours, at the end of which time it was diluted with water, made acidic with hydrochloric acid, and stirred for 10 minutes. The reaction mixture was then neutralized with saturated sodium hydrogen carbonate solution and extracted with 3 portions of methylene chloride. The extract was dried over magnesium sulfate, filtered, and concentrated. The residue was purified by silica gel column chromatography (5% methanol-chloroform) to recover 1.13 g of a product. A 0.30 g portion of this product was dissolved in 10 ml of methanol, 5 ml of 20% HCl/methanol was added, and the mixture was concentrated. The residue was recrystallized from methanol to provide 0.22 g of the title compound.

Example 4

N-Methyl-N-[(2RS-11bSR)-9-isobutoxy-1,3,4,6,7,11b-hexahydro-2H-benzo[a]quinolizin-2-yl]methanesulfonamide hydrochloride (Compound No. 10)

To a solution of 400 mg of the N-methyl-N-[(2RS,11bSR)-9-hydroxy-1,3,4,6,7,11b-hexahydro-2H-benzo[a]quinolizin-2-yl]methanesulfonamide obtained in Example 3 in 10 ml of N,N-dimethylformamide was added 57 mg of NaH, and the mixture was stirred for 15 minutes. To this reaction mixture was added 263 mg of isobutyl bromide dropwise, and the mixture was stirred at 60°C overnight. This reaction mixture was concentrated under reduced pressure and the residue was diluted with water and extracted with chloroform twice. The extract was dried over magnesium sulfate, filtered, and concentrated, and the residue was purified by silica gel column chromatography (2% methanol-chloroform) to give a light-yellow-oily substance. This compound was dissolved in 10 ml of methanol, 5 ml of 20% HCl/methanol was added, and the mixture was concentrated. The residue was recrystallized from methanol to provide 385 mg of the title compound.

Example 5

N,N-Dimethyl-N'-[(2RS-11bSR)-9-methoxy-1,3,4,6,7,11b-hexahydro-2H-benzo[a]quinolizin-2-yl]-N'-methylsulfamide hydrochloride (Compound No. 16)

To a solution of 3.0 g of the N-(9-methoxy-1,3,4,6,7,11b-hexahydro-2H-benzo[a]quinolizin-2-yl)-N-methylamine obtained in Reference Example 1 in 60 ml of methylene chloride was added 5.6 g of dimethylsulfamoyl chloride, followed by addition of 3.9 g of triethylamine, under ice-cooling and stirring, and the mixture was stirred at 40°C for 5 hours. This reaction mixture was diluted with water, made alkaline with 2N-NaOH, and extracted with chloroform three times. The extract was dried over magnesium sulfate, filtered, and concentrated to give a residue (a cis/trans mixture). This residue was purified by silica gel column chromatography (5% methanol-chloroform) to provide the cis-substance. This compound was dissolved in 10 ml of methanol, then 20 ml of 20% HCl/methanol was added, and the mixture was concentrated. The residue was recrystallized from methanol to provide 2.1 g of the title compound.

Example 6

N-Methyl-N-[(2RS,11bSR)-9-methoxy-1,3,4,6,7,11b-hexahydro-2H-benzo[a]quinolizin-2-yl]-1-pyrrolidinesulfonamide hydrochloride (Compound No. 17)

To a solution of 2.0 g of the N-(9-methoxy-1,3,4,6,7,11b-hexahydro-2H-benzo[a]quinolizin-2-yl)-N-methylamine obtained in Reference Example 1 in 50 ml of methylene chloride was added 2.8 g of 1-pyrrolidinesulfonyl chloride as prepared by the procedure described in the literature (S.K.Gupta, Synthesis, 1977, 39), followed by addition of 1.6 g of triethylamine, under ice-cooling, and the mixture was stirred at room temperature overnight. This reaction mixture was diluted with water, made alkaline with 2N-NaOH, and extracted with chloroform three times. The extract was dried over magnesium sulfate, filtered, and concentrated to give a residue (a cis/trans mixture). This residue was purified by silica gel column chromatography (5% methanol-chloroform) to provide the cis-substance. This compound was dissolved in 5 ml of methanol, then 10 ml of 20% HCl/methanol was added, and the mixture was concentrated. The residue

was recrystallized from methanol to provide 1.3 g of the title compound.

Example 7

N-Methyl-N-[(2R,11bS)-9-methoxy-1,3,4,6,7,11b-hexahydro-2H-benzo[a]quinolizin-2-yl]methanesulfonamide hydrochloride (Compound No. 18)

To a solution of 3.0 g of the N-[(2R,11bS)-9-methoxy-1,3,4,6,7,11b-hexahydro-2H-benzo[a]quinolizin-2-yl]-N-methylamine obtained in Reference Example 2 in 100 ml of methylene chloride was added 1.8 g of methanesulfonyl chloride, followed by addition of 2.6 g of triethylamine, under ice-cooling and stirring, and the mixture was stirred at room temperature for 1 hour. This reaction mixture was diluted with water, made alkaline with 2N-NaOH, and extracted with chloroform three times. The extract was dried over magnesium sulfate, filtered, and concentrated to give a residue [(2R,11bS)-substance:(2S,11bS)-substance = 9:1]. This residue was purified by silica gel column chromatography (5% methanol-chloroform) to provide N-methyl-N-[(2R,11bS)-9-methoxy-1,3,4,6,7,11b-hexahydro-2H-benzo[a]quinolizin-2-yl]methanesulfonamide.

$^1$H-NMR ($CDCl_3$) $\delta$ : 7.07 (1H, d, J=9 Hz), 6.73 (1H, dd, J=9 Hz, 3 Hz), 6.64 (1H, d, J=3 Hz), 4.03 (1H, m), 3.78 (3H, s), 3.3-2.9 (4H, m), 2.90 (3H, s), 2.81 (3H, s), 2.8-2.3 (4H, m), 2.1-1.5 (3H, m)

This compound was dissolved in 5 ml of methanol, then 10 ml of 20% HCl/methanol was added, and the mixture was concentrated to provide 1.7 g of the objective hydrochloride.

Specific rotation $[\alpha]_D^{25}$ = +2.38° (c=2.009 g/dl, $H_2O$)

Example 8

N-Methyl-N-[(2R,11bS)-9-methoxy-1-3-4-6-7-11b-hexahydro-2H-benzo[a]quinolizin-2-yl]-4-methoxybenzenesulfonamide hydrochloride (Compound No. 19)

To a solution of 1.1 g of the N-[(2R,11bS)-9-methoxy-1,3,4,6,7,11b-hexahydro-2H-benzo[a]quinolizin-2-yl]-N-methylamine obtained in Reference Example 2 in 40 ml of methylene chloride was added 1.1 g of 4-methoxybenzenesulfonyl chloride, followed by addition of 0.9 g of triethylamine, under ice-cooling and stirring, and the mixture was stirred at room temperature for 1 hour.

This reaction mixture was diluted with water, made alkaline with 2N-NaOH, and extracted with chloroform three times. The extract was dried over magnesium sulfate, filtered, and concentrated to give a residue [(2R,11bS)-substance:(2S,11bS)-substance = 9:1]. This residue was purified by silica gel column chromatography (5% methanol-chloroform) to provide the (2R,11bS)-substance. This compound was dissolved in 5 ml of methanol, then 10 ml of 20% HCl/methanol was added, and the mixture was concentrated. The residue was recrystallized from methanol to provide 0.7 g of the objective hydrochloride.

Specific rotation $[\alpha]_D^{25}$ = +21.37° (c=1.029 g/dl, $CH_3OH$)

Example 9

N-Methyl-N-[(2R,11bS)-9-methoxy-1,3,4,6,7,11b-hexahydro-2H-benzo[a]quinolizin-2-yl]ethanesulfonamide hydrochloride (Compound No. 20)

To a solution of 2.0 g of the N-[(2R,11bS)-9-methoxy-1,3,4,6,7,11b-hexahydro-2N-benzo[a]quinolizin-2-yl]-N-methylamine obtained in Reference Example 2 in 50 ml of methylene chloride was added 1.6 g of ethanesulfonyl chloride, followed by addition of 1.6 g of triethylamine, under ice-cooling and stirring, and the mixture was stirred at room temperature for 5 hours. This reaction mixture was diluted with water, made alkaline with 2N-NaOH, and extracted with chloroform three times. The extract was dried over magnesium sulfate, filtered, and concentrated. The resulting residue [(2R,11bS)-substance:(2S,11bS)-substance = 9:1] was purified by silica gel column chromatography (5% methanol-chloroform) to provide N-methyl-N-[(2R,11bS)-9-methoxy-1,3,4,6,7,11b-hexahydro-2N-benzo[a]quinolizin-2-yl]ethanesulfonamide.

$^1$H-NMR ($CDCl_3$) $\delta$ : 7.08 (1H, d, J=9 Hz), 6.73 (1H, dd, J=9 Hz, 3 Hz), 6.63 (1H, d, J=3 Hz), 3.97 (1H, m), 3.78 (3H, s), 3.3-2.8 (4H, m), 3.03 (2H, q, J=7 Hz), 2.82 (3H, s), 2.8-2.3 (4H, m), 2.1-1.5 (3H, m), 1.39 (3H,

t, J=7 Hz)

The above compound was dissolved in 5 ml of methanol, then 10 ml of 20% HCl/methanol was added, and the mixture was concentrated to provide 0.23 g of the objective hydrochloride.

Specific rotation $[\alpha]_D^{25}$ = +10.81° (c=1.054 g/dl, $H_2O$)

Example 10

N-Ethyl-N-[(2R,11bS)-9-methoxy-1,3,4,6,7,11b-hexahydro-2H-benzo[a]quinolizin-2-yl]methanesulfonamide hydrochloride (Compound No. 21)

To a solution of 4.0 g of the N-[(2R,11bS)-9-methoxy-1,3,4,6,7,11b-hexahydro-2H-benzo[a]quinolizin-2-yl]-N-ethylamine obtained in Reference Example 3 in 40 ml of methylene chloride was added 2.2 g of methanesulfonyl chloride, followed by addition of 3.3 g of triethylamine, under ice-cooling and stirring, and the mixture was stirred at room temperature for 2 hours. This reaction mixture was diluted with water, made alkaline with 2N-NaOH, and extracted with chloroform three times. The extract was dried over magnesium sulfate, filtered, and concentrated. The residue [(2R,11bS)-substance:(2S,11bS)-substance = 4:1] was purified by silica gel column chromatography (5% methanol-chloroform) to provide N-ethyl-N-[(2R,11bS)-9-methoxy-1,3,4,6,7,11b-hexahydro-2H-benzo[a]quinolizin-2-yl]methanesulfonamide.

$^1$H-NMR ($CDCl_3$) $\delta$ : 7.09 (1H, d, J=9 Hz), 6.73 (1H, dd, J=9 Hz, 3 Hz), 6.64 (1H, d, J=3 Hz), 3.93 (1H, m), 3.78 (3H, s), 3.4-2.9 (4H, m), 3.26 (2H, q, J=7 Hz), 2.93 (3H, s), 2.8-2.3 (4H, m), 2.1-1.5 (3H, m), 1.24 (3H, t, J=7 Hz)

The above compound was dissolved in 5 ml of methanol, then 10 ml of 20% HCl/methanol was added, and the mixture was concentrated to provide 1.6 g of the objective hydrochloride.

Specific rotation $[\alpha]_D^{25}$ = -8.46° (c=1.087 g/dl, $CH_3OH$)

Following the same procedure as Example 1, the compounds given Compound Nos. 2-5, 7, 8, 11-15, 22, and 23, respectively, below were produced. Those compounds as purified were invariably cis-substances [50:50 mixtures of [(2R,11bS)-substance and (2S,11bR)-substance].

(Compound No. 2) N-methyl-N-[(2RS,11bSR)-9-methoxy-1,3,4,6,7,11b-hexahydro-2H-benzo[a]quinolizin-2-yl]propanesulfonamide hydrochloride
(Compound No. 3) N-methyl-N-[(2RS,11bSR)-9-methoxy-1,3,4,6,7,11b-hexahydro-2H-benzo[a]quinolizin-2-yl]isopropanesulfonamide hydrochloride
(Compound No. 4) N-methyl-N-[(2RS,11bSR)-9-methoxy-1,3,4,6,7,11b-hexahydro-2H-benzo[a]quinolizin-2-yl]-4-chlorobenzenesulfonamide hydrochloride
(Compound No. 5) N-methyl-N-[(2RS,11bSR)-9-methoxy-1,3,4,6,7,11b-hexahydro-2H-benzo[a]quinolizin-2-yl]-2-hydroxyethanesulfonamide hydrochloride
(Compound No. 7) N-methyl-N-[(2RS,11bSR)-9-methoxy-1,3,4,6,7,11b-hexahydro-2H-benzo[a]quinolizin-2-yl]benzenesulfonamide hydrochloride
(Compound No. 8) N-(2-methoxyethyl)-N-[(2RS,11bSR)-9-methoxy-1,3,4,6,7,11b-hexahydro-2H-benzo[a]quinolizin-2-yl]methanesulfonamide hydrochloride
(Compound No. 11) N-isopropyl-N-[(2RS,11bSR)-9-methoxy-1,3,4,6,7,11b-hexahydro-2H-benzo[a]quinolizin-2-yl]methanesulfonamide hydrochloride
(Compound No. 12) N-methyl-N-[(2RS,11bSR)-9-methoxy-1,3,4,6,7,11b-hexahydro-2H-benzo[a]quinolizin-2-yl]-p-toluenesulfonamide hydrochloride
(Compound No. 13) N-methyl-N-[(2RS,11bSR)-9-methoxy-1,3,4,6,7,11b-hexahydro-2H-benzo[a]quinolizin-2-yl]-4-fluorobenzenesulfonamide hydrochloride
(Compound No. 14) N-methyl-N-[(2RS,11bSR)-9-methoxy-1,3,4,6,7,11b-hexahydro-2H-benzo[a]quinolizin-2-yl]-2,5-dimethoxybenzenesulfonamide hydrochloride
(Compound No. 15) N-methyl-N-[(2RS,11bSR)-9-methoxy-1,3,4,6,7,11b-hexahydro-2H-benzo[a]quinolizin-2-yl]-3,4-dimethoxybenzenesulfonamide hydrochloride
(Compound No. 22) N-methyl-N-[(2RS,11bSR)-9-methoxy-1,3,4,6,7,11b-hexahydro-2H-benzo[a]quinolizin-2-yl]ethanesulfonamide hydrochloride

(Compound No. 23) N-ethyl-N-[(2RS,11bSR)-9-methoxy-1,3,4,6,7,11b-hexahydro-2H-benzo[a]quinolizin-2-yl]methanesulfonamide hydrochloride

The structural formulas and physical constants of the compounds produced are presented in the following tables. When the compound is a cis-substance, only the structural formula of the (2R,11bS)-substance is shown.

Table 1

| Compound No. | Structural formula | m. p. (°C) State | Molecular formula Elemental analysis Calcd. (%) Found (%) |
|---|---|---|---|
| 1 | 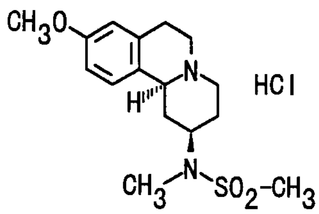 | 264 - 266 Colorless crystals | $C_{16}H_{24}N_2O_3S \cdot HCl \cdot 1/2H_2O$ C, 51.95; H, 7.08; N, 7.57 C, 52.14; H, 6.78; N, 7.59 |
| 2 | 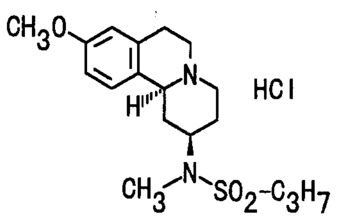 | 202 - 203 Colorless powder | $C_{18}H_{28}N_2O_3S \cdot HCl \cdot 1/4H_2O$ C, 54.95; H, 7.56; N, 7.12 C, 55.07; H, 7.38; N, 7.14 |
| 3 | 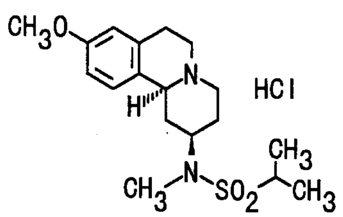 | 236 - 238 Light-yellow powder | $C_{18}H_{28}N_2O_3S \cdot HCl \cdot 1/4H_2O$ C, 54.95; H, 7.56; N, 7.12 C, 54.91; H, 7.37; N, 7.09 |
| 4 | 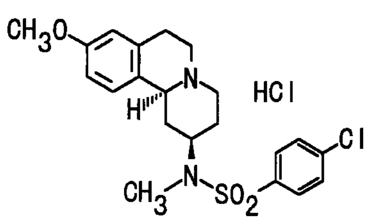 | 247 - 249 Colorless crystals | $C_{21}H_{25}ClN_2O_3S \cdot HCl \cdot 1/5H_2O$ C, 54.71; H, 5.77; N, 6.08 C, 54.72; H, 5.68; N, 6.09 |
| 5 | 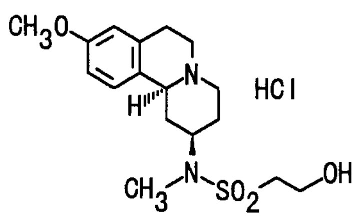 | 243 - 245 Colorless powder | $C_{17}H_{26}N_2O_4S \cdot HCl \cdot 1/4H_2O$ C, 51.64; H, 7.01; N, 7.08 C, 51.75; H, 7.05; N, 7.12 |

Table 2

| Compound No. | Structural formula | m. p. (°C) State | Molecular formula Elemental analysis Calcd. (%) Found (%) |
|---|---|---|---|
| 6 | 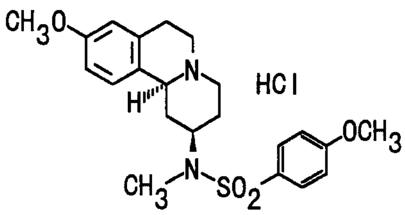 | 210 - 212 Colorless crystals | $C_{22}H_{28}N_2O_4S \cdot HCl \cdot 1/5H_2O$<br>C, 57.87; H, 6.49; N, 6.14<br>C, 57.95; H, 6.38; N, 6.26 |
| 7 | 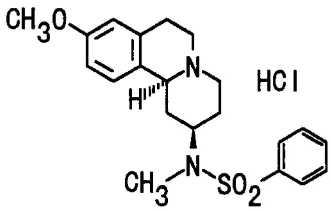 | 243 - 245 Light-yellow crystals | $C_{21}H_{26}N_2O_3S \cdot HCl \cdot 1/2H_2O$<br>C, 58.39; H, 6.53; N, 6.48<br>C, 58.34; H, 6.35; N, 6.38 |
| 8 | 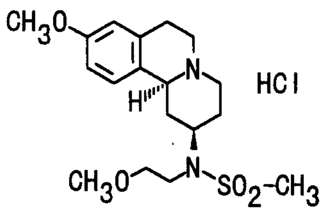 | 165 - 168 Colorless powder | $C_{18}H_{28}N_2O_4S \cdot HCl \cdot 1/2H_2O$<br>C, 52.23; H, 7.30; N, 6.77<br>C, 52.40; H, 7.33; N, 6.44 |
| 9 | 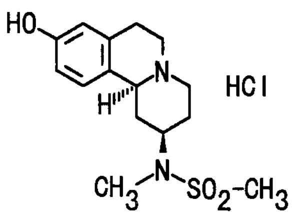 | 203 - 204 Colorless powder | $C_{15}H_{22}N_2O_3S \cdot HCl \cdot H_2O$<br>C, 49.37; H, 6.91; N, 7.68<br>C, 49.42; H, 6.68; N, 7.79 |
| 10 | 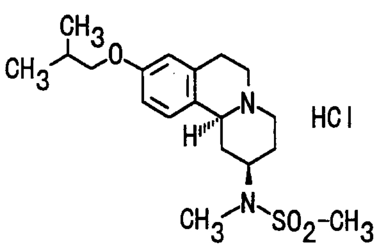 | 198 - 200 Colorless powder | $C_{19}H_{30}N_2O_3S \cdot HCl \cdot 1/4H_2O$<br>C, 56.00; H, 7.79; N, 6.87<br>C, 56.15; H, 7.62; N, 6.94 |

Table 3

| Compound No. | Structural formula | m. p. (°C) State | Molecular formula Elemental analysis Calcd. (%) Found (%) |
|---|---|---|---|
| 11 | 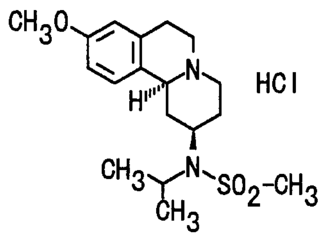 | 263 - 265 Colorless crystals | $C_{18}H_{28}N_2O_3S \cdot HCl \cdot 1/4H_2O$<br>C, 54.95; H, 7.56; N, 7.12<br>C, 54.80; H, 7.34; N, 7.08 |
| 12 | 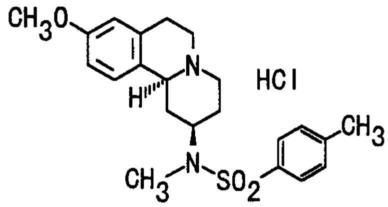 | 234 - 236 Colorless crystals | $C_{22}H_{28}N_2O_3S \cdot HCl \cdot 1/4H_2O$<br>C, 59.85; H, 6.73; N, 6.34<br>C, 59.72; H, 6.66; N, 6.38 |
| 13 | 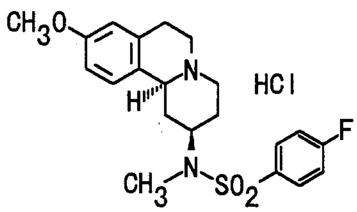 | 239 - 249 Colorless powder | $C_{21}H_{25}FN_2O_3S \cdot HCl \cdot 1/5H_2O$<br>C, 56.74; H, 5.99; N, 6.30<br>C, 56.61; H, 5.96; N, 6.45 |
| 14 | 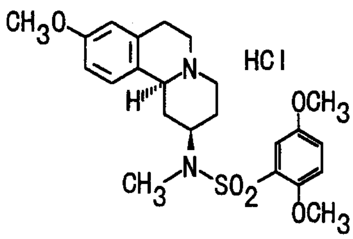 | 247 - 249 Colorless crystals | $C_{23}H_{30}N_2O_5S \cdot HCl \cdot 1/5H_2O$<br>C, 56.77; H, 6.50; N, 5.76<br>C, 56.79; H, 6.43; N, 5.73 |
| 15 | 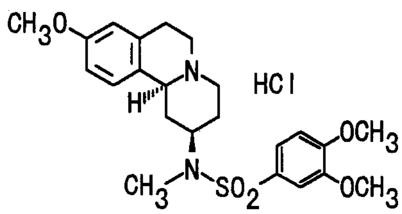 | 206 - 208 Colorless crystals | $C_{23}H_{30}N_2O_5S \cdot HCl \cdot 1/5H_2O$<br>C, 56.77; H, 6.50; N, 5.76<br>C, 56.96; H, 6.41; N, 5.78 |

Table 4

| Compound No. | Structural formula | m. p. (°C) State | Molecular formula Elemental analysis Calcd. (%) Found (%) |
|---|---|---|---|
| 16 | 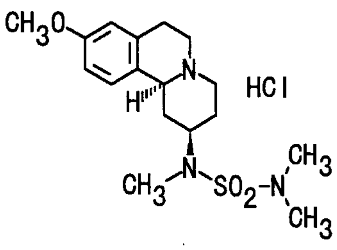 | 251 - 252 (decomposition) Colorless crystals | $C_{17}H_{27}N_3O_3S \cdot HCl$ C, 52.36; H, 7.24; N, 10.78 C, 52.15; H, 7.03; N, 10.69 |
| 17 | 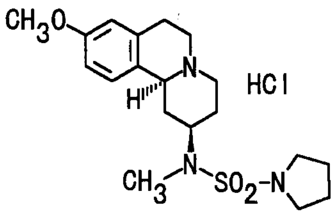 | 231 - 233 Colorless powder | $C_{19}H_{29}N_3O_3S \cdot HCl$ C, 54.86; H, 7.27; N, 10.10 C, 54.56; H, 7.15; N, 9.94 |
| 18 | 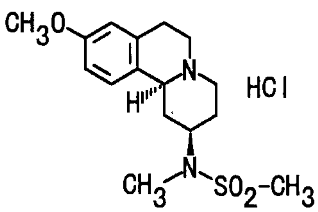 | Colorless amorphous | $C_{16}H_{24}N_2O_3S \cdot HCl$ C, 53.25; H, 6.98; N, 7.76 C, 53.13; H, 6.86; N, 7.71 |
| 19 | 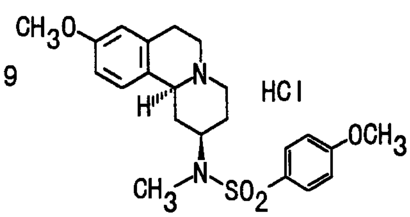 | 231 - 233 (decomposition) Colorless crystals | $C_{22}H_{28}N_2O_4S \cdot HCl$ C, 58.33; H, 6.45; N, 6.18 C, 58.21; H, 6.47; N, 6.13 |
| 20 | 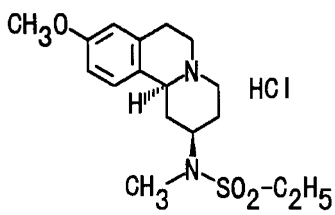 | Colorless amorphous | $C_{17}H_{26}N_2O_3S \cdot HCl$ C, 54.46; H, 7.26; N, 7.47 C, 54.22; H, 7.21; N, 7.39 |

Table 5

| Compound No. | Structural formula | m. p. (°C) State | Molecular formula Elemental analysis Calcd. (%) Found (%) |
|---|---|---|---|
| 21 | 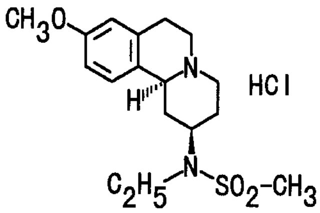 HCl | Colorless amorphous | $C_{17}H_{26}N_2O_3S \cdot HCl \cdot 1/5H_2O$<br>C, 53.94; H, 7.30; N, 7.40<br>C, 54.07; H, 7.23; N, 7.39 |
| 22 | 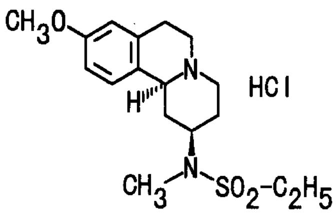 HCl | 242 - 243 (decomposition) Colorless powder | $C_{17}H_{26}N_2O_3S \cdot HCl$<br>C, 54.46; H, 7.26; N, 7.47<br>C, 54.40; H, 7.22; N, 7.52 |
| 23 | 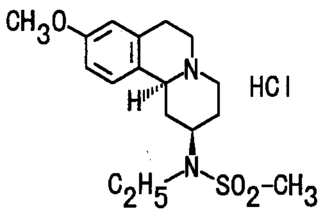 HCl | 231 - 234 Colorless crystals | $C_{17}H_{26}N_2O_3S \cdot HCl \cdot 1/2H_2O$<br>C, 53.18; H, 7.35; N, 7.30<br>C, 53.32; H, 7.08; N, 7.38 |

The following are the results of pharmacological tests which demonstrate the usefulness of some representative species of the compound of the invention.

Test Example 1

Effect on an IBS model (1)

Six-week-old male SD rats, non-fasted, were used in groups of 8 individuals. In accordance with the method of Williams et al. (Gastroenterology, 1988; 94:611-621), animals in the control group and treatment groups were loaded with a wrap-restraint stress while those in the normal group were not loaded and weight (g) of fecal pellets in the subsequent one-hour period was determined. In the treatment groups, a predetermined dose of the corresponding test drug was administered subcutaneously 30 minutes before stress loading. The inhibition rate (%) was calculated by means of the following equation.

$$\text{Inhibition rate} = [(C-D)/(C-N)] \times 100$$

where C represents the weight of the fecal pellets in the control group, D represents the weight of the fecal pellets in the treatment group, and N represents the weight of the fecal pellets in the normal group.

Statistical significance of the treatment group versus the control group was tested by Dunnett's multiple comparison. The results are shown in Table 6.

Table 6

| Test drug | Dosage (mg/kg) | Inhibition (%) vs. control |
|---|---|---|
| Compound No. 1 | 3 | 97.7** |
| Compound No. 6 | 3 | 86.2** |
| Compound No. 7 | 3 | 76.6** |
| Compound No. 13 | 3 | 72.4** |
| Compound No. 14 | 3 | 75.6** |
| Compound No. 18 | 3 | 96.6** |

**P<0.01

The compound of the invention significantly inhibited the stress-induced enhancement of defecation. The effectiveness of the compound as a therapeutic drug for IBS is thus evident.

Test Example 2

<u>Effect on an IBS model (2)</u>

Six-week-old male SD rats, non-fasted, were used in groups of 8 individuals. In accordance with the method of Williams et al. (Gastroenterology, 1988; 94:611-621), animals in the control group and treatment groups were loaded with a wrap-restraint stress while those in the normal group were not loaded and weight (g) of the fecal pellets in the subsequent one-hour period was determined. In the treatment groups, a predetermined dose of the corresponding test drug was administered orally 60 minutes before stress loading. As reference control drugs, N-methyl-N-[(2RS,11bSR)-1,3,4,6,7,11b-hexahydro-2H-benzo[a]quinolizin-2-yl]propanesulfonamide hydrochloride (hereinafter referred to as Compound A) and N-(2-methanesulfonamidoethyl)-N-[(2RS,11bSR)-1,3,4,6,7,11b-hexahydro-2H-benzo[a]quinolizin-2-yl]benzenesulfonamide hydrochloride (hereinafter referred to as compound B), both of which are described in Japanese Laid-open S62-67031 as having gastrointestinal peristalsis-modulating activity were used. The inhibition rate (%) was calculated by means of the following equation.

$$\text{Inhibition rate} = [(C-D)/(C-N)] \times 100$$

where C represents the weight of the fecal pellets in the control group, D represents the weight of the fecal pellets in the treatment group, and N represents the weight of the fecal pellets in the normal group.

Statistical significance of the treatment group versus the control group was tested by Dunnett's multiple comparison.

The results are shown in Table 7.

Table 7

| Test drug | Dosage (mg/kg) | Inhibition (%) vs. control |
|---|---|---|
| Compound No. 1 | 10 | 53.2* |
| | 30 | 96.6** |
| Compound No. 16 | 10 | 73.3** |
| | 30 | 91.7** |
| Compound No. 18 | 10 | 91.1** |
| Compound No. 20 | 10 | 69.4** |
| | 30 | 88.9** |
| Compound No. 21 | 10 | 64.6** |
| | 30 | 92.6** |
| Compound A | 10 | -1.2 |
| | 30 | 1.4 |
| Compound B | 10 | -4.7 |
| | 30 | -30.0 |

*P<0.05,
**P<0.01

Test Example 3

<u>Effect on an IBS model (3)</u>

Six-week-old male SD rats were used in groups of 8 individuals. In accordance with the method of Enck et al. (Gut, 1989; 30:455-459), animals in the control group and treatment groups were loaded with water avoidance stress, while those in the normal groups were not loaded and the number of fecal pellets during the subsequent one-hour period was determined. In the treatment groups, a predetermined dose of the corresponding test drug was administered orally 60 minutes before stress loading. As reference control drugs, Compound A and Compound B mentioned above were used. The inhibition rate (%) was calculated by means of the following equation.

$$\text{Inhibition rate (\%)} = [(NC-ND)/(NC-NN)] \times 100$$

where NC represents the number of the fecal pellets in the control group, ND represents the number of the fecal pellets in the treatment group, and NN represents the number of the fecal pellets in the normal group.

Statistical significance of the treatment group versus the control group was tested by Dunnett's multiple comparison.

The results are shown in Table 8.

Table 8

| Test drug | Dosage (mg/kg) | Inhibition (%) vs. control |
|---|---|---|
| Compound No. 1 | 30 | 81.8* |
| Compound No. 18 | 3 | 72.4* |
| Compound No. 18 | 10 | 82.9** |
| Compound A | 30 | -20.7 |
| Compound B | 30 | -58.3 |

*P<0.05,
**P<0.01

It is apparent from Test Examples 2 and 3 that the compound of the invention remarkably inhibited defecation at the dose levels at which Compound A and Compound B, both of which are described in Japanese Laid-open S62-67031 as having gastrointestinal peristalsis-modulating activity, did not inhibit defecation accelerated by stress. Therefore, it is clear that the compound of the invention is more useful than the benzoquinolizine derivative according to Japanese Laid-open S62-67031 as a therapeutic drug for IBS.

Test Example 4

Acute toxicity test

Six-week-old male ddY mice were used in groups of 5 individuals. The animals were fasted from the previous day (16-18 hours before the experiment) and a predetermined dose of the compound of the invention was administered orally. The number of deaths during the subsequent one-week period was investigated.
The results are shown in Table 9.

Table 9

| Test drug | Dosage (mg/kg) | Dead/Total |
|---|---|---|
| Compound No. 1 | 100 | 0/5 |
| Compound No. 6 | 100 | 0/5 |
| Compound No. 16 | 300 | 0/5 |
| Compound No. 18 | 250 | 0/5 |
| Compound No. 21 | 250 | 0/5 |

Thus, no death was encountered, nor was observed any abnormal finding.

Formulation Example 1

Tablets (oral tablets)

In 200 mg per tablet

| | |
|---|---|
| Compound No. 1 | 20 mg |
| Corn starch | 88 mg |
| Crystalline cellulose | 80 mg |

(continued)

| | |
|---|---|
| Carboxymethylcellulose calcium | 10 mg |
| Light silicic anhydride | 1 mg |
| Magnesium stearate | 1 mg |

The mixed powder of the above formulation is compressed to provide oral tablets.

Formulation Example 2

Tablets (oral tablets)

In 200 mg per tablet

| | |
|---|---|
| Compound No. 6 | 20 mg |
| Corn starch | 88 mg |
| Crystalline cellulose | 80 mg |
| Carboxymethylcellulose calcium | 10 mg |
| Light silicic anhydride | 1 mg |
| Magnesium stearate | 1 mg |

The mixed powder of the above formulation is compressed to provide oral tablets.

INDUSTRIAL APPLICABILITY

Thus, the compound of the invention is useful as a therapeutic drug for irritable bowel syndrome.

**Claims**

1. A benzoquinolizine derivative of the following formula [1] or a pharmaceutically acceptable salt thereof,

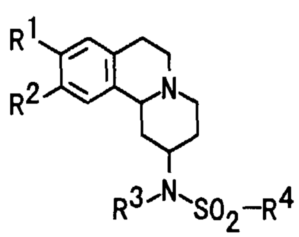

[1]

wherein

$R^1$ represents hydroxy or alkoxy;
$R^2$ represents hydrogen, hydroxy or alkoxy;
$R^3$ represents alkyl: the said alkyl is optionally substituted by hydroxy, alkoxy, amino, monoalkylamino, or dialkylamino;
$R^4$ represents alkyl (the said alkyl is optionally substituted by hydroxy or alkoxy), aryl (the said aryl is optionally substituted by alkyl, halogen, hydroxy, alkoxy, amino, monoalkylamino, or dialkylamino), or $-NR^5R^6$; $R^5$ and $R^6$ are the same or different and each represents alkyl, or $R^5$ and $R^6$ taken together with the adjacent N atom represent a 5-membered or 6-membered cyclic amino group.

2. The benzoquinolizine derivative or pharmaceutically acceptable salt according to Claim 1 wherein the steric relationship between the hydrogen atom at 2-position and the hydrogen atom at 11b-position is cis; $R^1$ is alkoxy, $R^2$ is hydrogen; $R^3$ is unsubstituted alkyl; $R^4$ is (1) unsubstituted alkyl, (2) aryl which is optionally substituted by halogen or alkoxy, or (3) dialkylamino.

3. The benzoquinolizine derivative or pharmaceutically acceptable salt according to Claim 1 wherein the compound is selected from the group consisting of the following compounds (1) to (8):

(1) N-methyl-N-[(2RS,11bSR)-9-methoxy-1,3,4,6,7,11b-hexahydro-2H-benzo[a]quinolizin-2-yl]methanesulfonamide
(2) N-methyl-N-[(2RS,11bSR)-9-methoxy-1,3,4,6,7,11b-hexahydro-2H-benzo[a]quinolizin-2-yl]-4-methoxybenzenesulfonamide
(3) N-methyl-N-[(2RS,11bSR)-9-methoxy-1,3,4,6,7,11b-hexahydro-2H-benzo[a]quinolizin-2-yl]benzenesulfonamide
(4) N-methyl-N-[(2RS,11bSR)-9-methoxy-1,3,4,6,7,11b-hexahydro-2H-benzo[a]quinolizin-2-yl]-4-fluorobenzensulfonamide
(5) N-methyl-N-[(2RS,11bSR)-9-methoxy-1,3,4,6,7,11b-hexahydro-2H-benzo[a]quinolizin-2-yl]-2,5-dimethoxybenzenesulfonamide
(6) N,N-dimethyl-N'-[(2RS,11bSR)-9-methoxy-1,3,4,6,7,11b-hexahydro-2H-benzo[a]quinolizin-2-yl]-N'-methylsulfamide
(7) N-methyl-N-[(2RS,11bSR)-9-methoxy-1,3,4,6,7,11b-hexahydro-2H-benzo[a]quinolizin-2-yl]ethanesulfonamide, and
(8) N-ethyl-N-[(2RS,11bSR)-9-methoxy-1,3,4,6,7,11b-hexahydro-2H-benzo[a]quinolizin-2-yl]methanesulfonamide

4. A gastrointestinal motility-modulating pharmaceutical composition comprising the compound described in Claims 1 to 3 as an active ingredient.

5. A pharmaceutical composition for the treatment of irritable bowel syndrome which comprises the compound described in Claims 1 to 3 as an active ingredient.

**INTERNATIONAL SEARCH REPORT**

International application No.
PCT/JP97/01320

A. CLASSIFICATION OF SUBJECT MATTER

Int. $Cl^6$ C07D455/04, A61K31/435

According to International Patent Classification (IPC) or to both national classification and IPC

B. FIELDS SEARCHED

Minimum documentation searched (classification system followed by classification symbols)

Int. $Cl^6$ C07D455/04, A61K31/435

Documentation searched other than minimum documentation to the extent that such documents are included in the fields searched

Electronic data base consulted during the international search (name of data base and, where practicable, search terms used)

CAS Online

C. DOCUMENTS CONSIDERED TO BE RELEVANT

| Category* | Citation of document, with indication, where appropriate, of the relevant passages | Relevant to claim No. |
|---|---|---|
| A | JP, 62-67031, A (Merck & Co., Inc.), March 26, 1987 (26. 03. 87), Claim etc. & EP, 216247, A & US, 4673680, A | 1 - 5 |
| A | JP, 60-500911, A (John Wyeth & Brother Ltd.), June 20, 1985 (20. 06. 85), Claim etc. & GB, 2136804, A & EP, 120637, A & WO, 84/03702, A & US, 4526967, A | 1 - 5 |
| A | US, 4481200, A (John Wyeth & Brother Ltd.), November 6, 1984 (06. 11. 84), Claim etc. & GB, 2117379, A | 1 - 5 |
| A | US, 4183937, A (John Wyeth & Brother Ltd.), January 15, 1980 (15. 01. 80), Claim etc. & GB, 1577187, A | 1 - 5 |
| A | US, 4076820, A (John Wyeth & Brother Ltd.), February 28, 1978 (28. 02. 78), Claim etc. & GB, 1513824, A | 1 - 5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| July 15, 1997 (15. 07. 97) | July 23, 1997 (23. 07. 97) |
| Name and mailing address of the ISA/ Japanese Patent Office | Authorized officer |
| Facsimile No. | Telephone No. |

Form PCT/ISA/210 (second sheet) (July 1992)